(19) Europäisches Patentamt
European Patent Office
Office européen des brevets

(11) Veröffentlichungsnummer: **0 459 200 A2**

(12) **EUROPÄISCHE PATENTANMELDUNG**

(21) Anmeldenummer: **91107670.1**

(22) Anmeldetag: **11.05.91**

(51) Int. Cl.5: **C07D 487/22**, A61K 31/40, //(C07D487/22,257:00,209:00, 209:00,209:00,209:00)

(30) Priorität: **21.05.90 DE 4016299**

(43) Veröffentlichungstag der Anmeldung: **04.12.91 Patentblatt 91/49**

(84) Benannte Vertragsstaaten: **CH DE FR GB IT LI**

(71) Anmelder: **BASF Aktiengesellschaft**
**Carl-Bosch-Strasse 38**
**W-6700 Ludwigshafen(DE)**

(72) Erfinder: **Montforts, Franz-Peter, Prof. Dr.**
**Beim Spieker 45**
**W-2804 Lilienthal(DE)**
Erfinder: **Haake, Gerold**
**Innsbrucker Strasse 92**
**W-2800 Bremen 1(DE)**
Erfinder: **Meier, Axel**
**Diedrich-Wilkens-Strasse 94**
**W-2800 Bremen 44(DE)**

(54) **Chlorinderivate.**

(57) Chlorinderivate der Formel

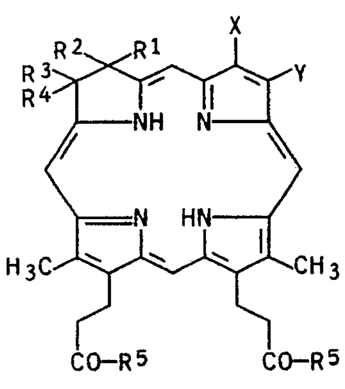

in der
eines der beiden Restepaare $R^1/R^2$ und $R^3/R^4$ Wasserstoff und $C_3$-$C_{12}$-Alkyl und das andere Methyl und $CH_2$-CO-$R^6$,
$R^5$ und $R^6$ unabhängig voneinander Hydroxy, $C_1$-$C_6$-Alkoxy, Amino oder $C_1$-$C_6$-Mono- oder Dialkylamino und einer der beiden Reste X und Y Wasserstoff und der andere Methyl bedeuten.

EP 0 459 200 A2

Die vorliegende Erfindung betrifft neue Chlorinderivate der Formel I

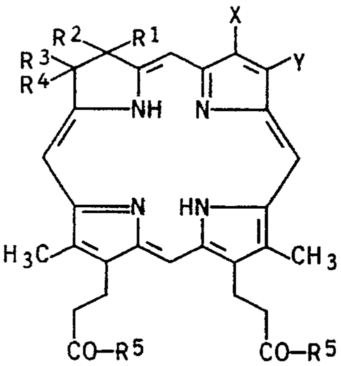

(I),

in der

eines der beiden Restepaare $R^1/R^2$ und $R^3/R^4$ Wasserstoff und $C_3$-$C_{12}$-Alkyl und das andere Methyl und $CH_2$-CO-$R^6$,

$R^5$ und $R^6$ gleich oder verschieden sind und unabhängig voneinander jeweils Hydroxy, $C_1$-$C_6$-Alkoxy, Amino oder $C_1$-$C_6$-Mono- oder Dialkylamino und

einer der beiden Reste X und Y Wasserstoff und der andere Methyl bedeuten.

Aus Angew. Chem. Band 98, Seiten 451 bis 452, 1986, ist bereits ein Chlorinderivat mit Ethylsubstituent bekannt, dem folgende Struktur zukommt

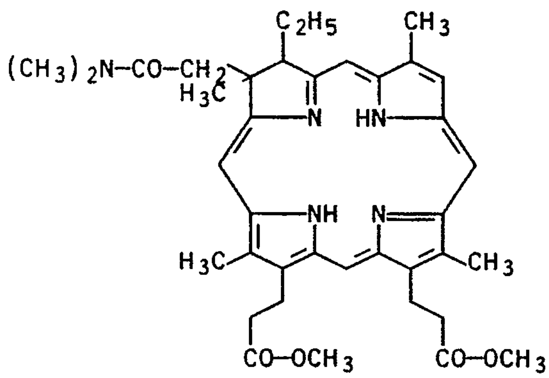

Es hat sich jedoch gezeigt, daß diese Verbindung nur über einen unzureichenden n-Octanol-Wasser-Verteilungskoeffizienten verfügt.

Aufgabe der vorliegenden Erfindung war es nun, neue Chlorinderivate bereitzustellen, die diesen Nachteil nicht mehr aufweisen.

Demgemäß wurden die eingangs näher definierten Chlorinderivate der Formel I gefunden.

Bei den neuen Chloriderivaten handelt es sich um Dihydroporphyrine, ihnen kommt das folgende Grundgerüst zu

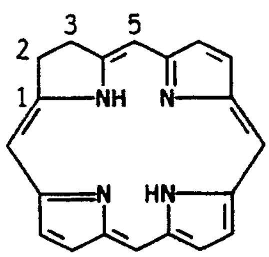

Die neuen Chlorinderivate der Formel I weisen an der Ringposition 2 und 3 jeweils ein Chiralitätszentrum auf, das entweder R- oder S-Konfiguration besitzt, wobei alle diese Verbindungen vom Patentanspruch umfaßt werden (RR, SR, RS und SS).

Alle in der obengenannten Formel I auftretenden Alkylgruppen können sowohl geradkettig als auch verzweigt sein.

Geeignete $C_3$-$C_{12}$-Alkylgruppen in Formel I sind z.B. Propyl, Isopropyl, Butyl, Isobutyl, sec-Butyl, Pentyl, Isopentyl, Neopentyl, tert-Pentyl, Hexyl, 2-Methylpentyl, Heptyl, 2-Methylhexyl, Octyl, 2-Ethylhexyl, Nonyl, Decyl, Undecyl oder Dodecyl.

Reste $R^5$ und $R^6$ sind z.B. Methoxy, Ethoxy, Propoxy, Isopropoxy, Butoxy, Isobutoxy, sec-Butoxy, Pentyloxy, Isopentyloxy, Neopentyloxy, Hexyloxy, 2-Methylpentyloxy, Mono- oder Dimethylamino, Mono- oder Diethylamino, Mono- oder Dipropylamino, Mono- oder Diisopropylamino, Mono- oder Dibutylamino, Mono- oder Diisobutylamino, Mono- oder Dipentylamino, Mono- oder Dihexylamino oder N-Methyl-N-ethylamino.

Hervorzuheben sind Chlorinderivate, die der Formel Ia

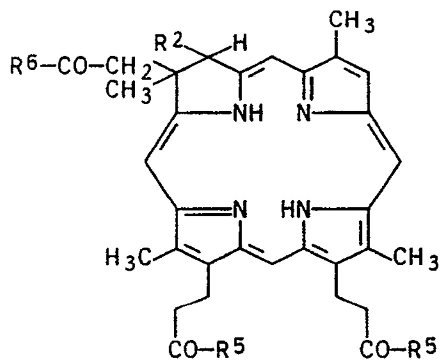

(Ia)

entsprechen, in der

$R^2$ $C_3$-$C_{12}$-Alkyl und

$R^5$ und $R^6$ gleich oder verschieden sind und unabhängig voneinander jeweils Hydroxy, $C_1$-$C_6$-Alkoxy, Amino oder $C_1$-$C_6$-Mono- oder Dialkylamino

bedeuten.

Bevorzugt sind Chlorinderivate der Formel Ia, in der

$R^2$ $C_5$-$C_{12}$-Alkyl und

$R^2$ und $R^3$ unabhängig voneinander jeweils Hydroxy, $C_1$-$C_4$-Alkoxy oder $C_1$-$C_4$-Dialkylamino

bedeuten.

Besonders bevorzugt sind Chlorinderivate der Formel Ia, in der $R^6$ $C_1$-$C_2$-Dialkylamino, insbesondere Dimethylamino, und $R^5$ Hydroxy oder $C_1$-$C_4$-Alkoxy bedeuten.

Insbesondere zu nennen sind Chlorinderivate der Formel Ia, in der $R^2$ $C_6$-$C_{10}$-Alkyl bedeutet, wobei Chlorinderivate der Formel Ia, in der $R^2$ Heptyl bedeutet, besonders hervorzuheben sind.

Weiterhin insbesondere zu nennen sind Chlorinderivate der Formel Ia, in der $R^6$ $C_1$-$C_2$-Dialkylamino, insbesondere Dimethylamino, und $R^5$ jeweils Hydroxy bedeuten.

Die Herstellung der neuen Chlorinderivate kann nach an sich bekannten Methoden, wie sie beispielsweise in Angew. Chem. Band 98, Seiten 451 und 452, 1986, beschrieben sind, erfolgen.

So kann man z.B. Deuteroporphyrindimethylester der Formel II

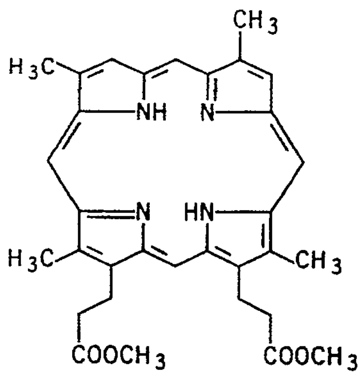

(II),

der auf an sich bekannte Weise aus Häminchlorid erhalten werden kann, in Form seines Kupferkomplexes mit einem Acylierungsmittel, beispielsweise mit einem Carbonsäureanhydrid der Formel III

L-CO-O-CO-L (III),

worin L jeweils $C_2$-$C_{11}$-Alkyl bedeutet, umsetzen und das resultierende Acylierungsprodukt der Formel IV

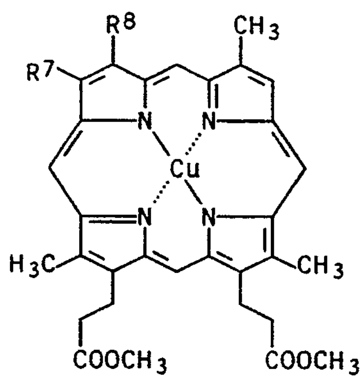

(IV),

in der einer der beiden Rest $R^7$ und $R^8$ Methyl und der andere $C_3$-$C_{12}$-Alkanoyl bedeuten, nach Entkupferung in die entsprechende Hydroxylalkylverbindung der Formel V

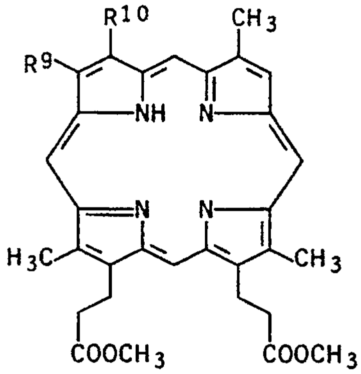

(V),

in der einer der beiden Reste $R^9$ und $R^{10}$ Methyl und der andere 1-Hydroxy-$C_3$-$C_{12}$-Alkyl bedeuten, umwandeln.

Aus der Hydroxyalkylverbindung V kann man anschließend, z.B. durch Umsetzung mit N,N-Dimethylacetamid-dimethylacetal, die Alkylidenverbindung der Formel VI

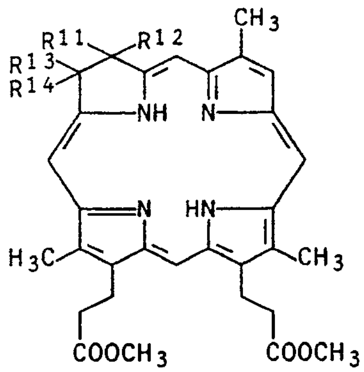

(VI),

in der eines der beiden Restpaare $R^{11}/R^{12}$ und $R^{13}/R^{14}$ $C_3$-$C_{12}$-Alkylid-1-en und das andere Methyl und $CH_2$-$CON(CH_3)_2$ bedeutet, herstellen und diese durch Hydrierung in die erfindungsgemäßen Chlorinderivate der Formel I überführen.

Die erfindungsgemäßen Chlorinderivate der Formel I können bei der photodynamischen Tumortherapie zur Anwendung kommen. Diese Therapie ist an sich bekannt und z.B. in T.J. Dougherty "Methods in Porphyrin Photosensitization", Ed. D. Kessel, Seiten 313-328 (Photodynamic therapy), 1985, Plenum, New York; J. Natl. Cancer Inst. Band 26, Seite 1 bis 8, 1961; Cancer Res. Band 38, Seiten 2628 bis 2635, 1978; oder J. Natl. Cancer Inst. Band 62, Seiten 231 bis 237, 1979, beschrieben.

Ein wichtiger Maßstab für die photodynamische Aktivität einer Verbindung, im Vergleich zu der bekannten Porphyrinpräparation, die als "Hämatoporphyrin-Derivat" bezeichnet und deren Anwendung in der photodynamischen Tumortherapie zur Zeit klinisch getestet wird, ist dabei der Lipid-Wasser-Verteilungskoeffizient, der entweder direkt bestimmt oder über die Retentionszeit mittels der Reversed-Phase-HPLC-Methode ermittelt werden kann (Cancer Letters Band 34, Seiten 283 bis 289, 1987).

Im vorliegenden Fall erfolgte die Ermittlung des n-Octanol-Wasser-Verteilungskoeffizienten gemäß EEC Directive 79/831, Annex V, Partition coefficient.

Dabei zeigte sich, daß die erfindungsgemäßen Verbindungen über einen höheren n-Octanol-Wasser-Verteilungskoeffizienten verfügen als die eingangs erwähnte Ethylverbindung. Der entsprechende Vergleichswert des "Hämatoporphyrin-Derivats" wird von den neuen Verbindungen I erreicht. Im Vergleich zum "Hämatoporphyrin-Derivat" besitzen die erfindungsgemäßen Chlorinderivate den Vorteil, daß sie einheitliche Verbindungen darstellen. Außerdem weisen sie ein längerwelliges Absorptionsmaximum mit höherem Extinktionskoeffizienten auf, was bei der photodynamischen Anwendung eine größere Eindringtiefe in das zu behandelnde Gewebe gewährleistet.

Die folgenden Beispiele sollen die Erfindung näher erläutern.

Beispiel 1

(Herstellung des Kupferkomplexes der Verbindung der Formel II)

Zu einer Lösung von 1,50 g (2,78 mmol) Deuteroporphyrindimethylester (Formel II) in 200 ml Chloroform gab man eine heiße Lösung von 0,60 g (3,0 mmol) Kupfer(II)-acetat-Monohydrat in 300 ml Methanol. Es wurde eine Stunde unter Rückfluß erhitzt und nach dem Abkühlen auf Raumtemperatur mit 300 ml Wasser verdünnt. Man extrahierte erschöpfend mit Dichlormethan und wusch die organischen Extrakte dreimal mit je 300 ml Wasser. Die vereinigten organischen Phasen wurden über Watte filtriert und am Rotationsverdampfer vom Lösungsmittel befreit. Der tiefrote Rückstand wurde durch Umkristallisation aus Chloroform/Methanol gereinigt. Man erhielt 1,60 g. (96 % d. Th.) des Kupferkomplexes. Fp.: 236 °C

Beispiel 2

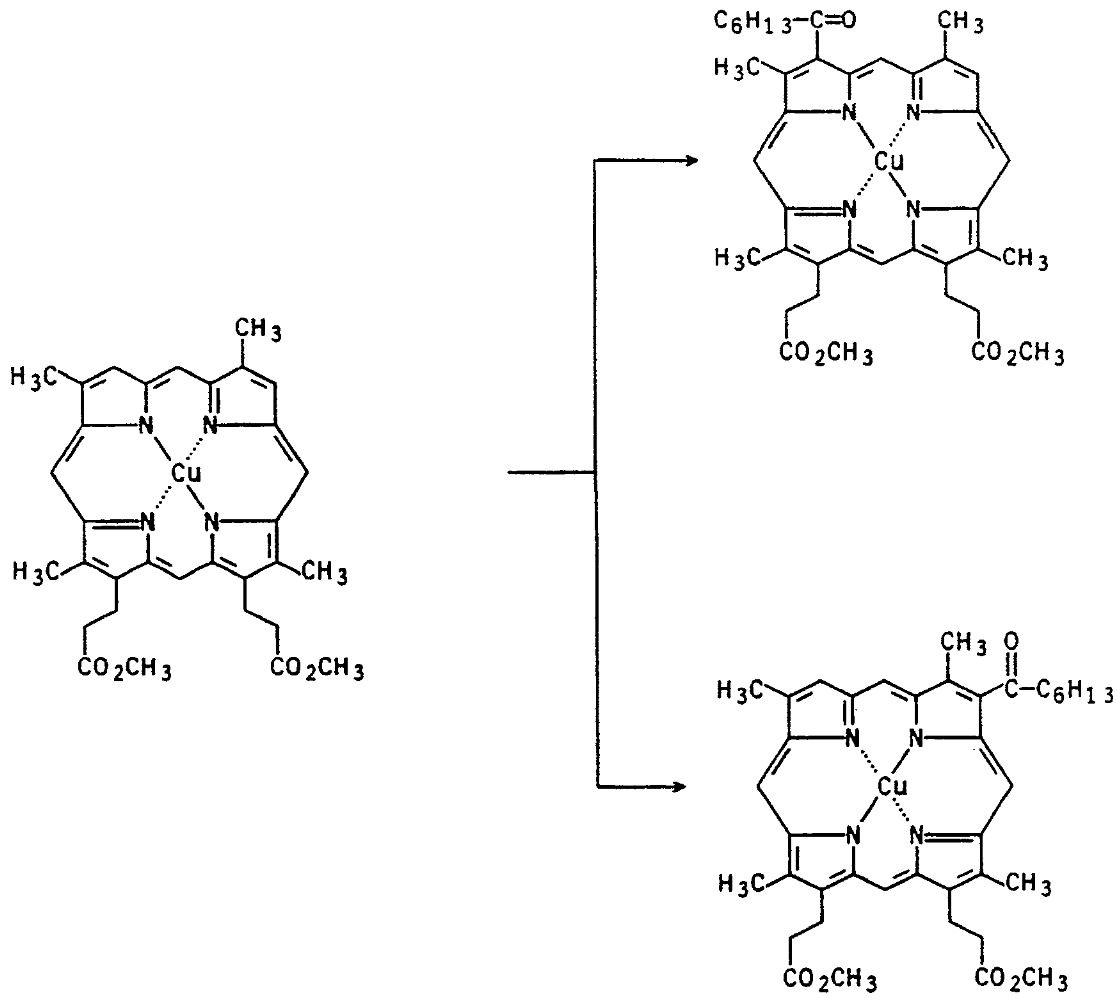

750 mg (1,250 mmol) Kupfer-deuteroporphyrindimethylester (aus Beispiel 1) wurden in 230 ml Dichlormethan gelöst und mit 15 ml Heptansäureanhydrid versetzt. Anschließend wurde die Lösung im Kryostaten auf -15 °C abgekühlt und binnen 25 Minuten mit einer auf -20 °C vorgekühlten Lösung von 1,1 ml Zinntetrachlorid in 160 ml Dichlormethan unter Rühren so versetzt, daß die Temperatur des Kühlmittels auf -10,5 °C steigen konnte. Hiernach rührte man noch 20 Minuten bei -10 bis -13 °C. Zu der grünen Suspension wurden ca. 300 ml Wasser gegeben, die organische Phase abgetrennt und die wäßrige Phase erschöpfend mit Dichlormethan extrahiert. Die vereinigten organischen Extrakte wurden zunächst zweimal mit ca. 200 ml Wasser, dann dreimal mit jeweils ca. 200 ml gesättigter Natriumhydrogencarbonatlösung und schließlich zweimal mit je ca. 200 ml gesättigter Natriumchloridlösung ausgeschüttelt. Die vereinigten organischen Phasen wurden über Watte filtriert und am Rotationsverdampfer vom Lösungsmittel befreit. Danach wurde das überschüssige Heptansäureanhydrid durch Kugelrohrdestillation im Ölpumpenvakuum bei 130± 10 °C entfernt. Das Rohprodukt wurde mittels Säulenchromatographie über neutrales Aluminiumoxid (Aktivität II-III) mit Dichlormethan:Petrolether 24:1 aufgearbeitet. Man erhielt 620 mg des gewünschten Kupfer-mono-(1-heptan-1-oyl)-deuteroporphyrindimethylesters als Gemisch der Isomeren (0,870 mmol, 69,6 % d. Th.).
UV/VIS (Chloroform): $\lambda_{max}(\epsilon)$ = 577 nm (18400), 532 nm (10700), 406 nm (186100), 321 nm (18000).

Beispiel 3

(Entkupferung des Kupferkomplexes aus Beispiel 2)

520 mg (0,730 mmol) Kupfer-heptan-1-oyl-deuteroporphyrindimethylester (Isomerengemisch) wurden 1 Stunde bei Raumtemperatur mit 5 ml konzentrierter Schwefelsäure in einer Argonatmosphäre behandelt. Das grüne Reaktionsgemisch wurde auf Eis gegossen und der braunviolette Rückstand erschöpfend mit

Dichlormethan extrahiert. Die vereinigten organischen Phasen wurden durch Filtration über Watte getrocknet und am Rotationsverdampfer vom Lösungsmittel befreit. Anschließend versetzte man den Rückstand mit etherischer Diazomethanlösung. Nach dreißigminütigem Rühren bei Raumtemperatur entfernte man überschüssiges Diazomethan sowie Lösungsmittel am Rotationsverdampfer. Das Rohprodukt wurde zunächst mittels Flash-Chromatographie (stationäre Phase: Matrex-Kieselgel, mobile Phase: Dichlormethan:Methanol 99:1) gereinigt. Es schloß sich eine zweite Flash-Chromatographie über Matrex-Kieselgel mit Dichlormethan:Essigsäureethylester 24:1 an, durch die Spuren verkupferten Ausgangsmaterials abgetrennt werden konnten. Ausbeute: 470 mg (99 % d. Th.)

UV/VIS (Chloroform):$\lambda_{max}(\epsilon)$ = 630 nm (1400), 574 nm (6400), 544 nm (10200), 506 nm (9700), 406 nm (163500).

Beispiel 4

Reduktion der Ketogruppe zur Hydroxygruppe im Isomerengemisch aus Beispiel 3)

500 mg (0,768 mmol) Heptan-1-oyl-deuteroporphyrindimethylester(Isomerengemisch) wurden in 150 ml Dichlormethan gelöst und mit einer auf -20° C vorgekühlten Lösung von 900 mg Natriumborhydrid in 50 ml frisch getrocknetem Methanol versetzt. Man ließ 18 Minuten bei Raumtemperatur unter einer Argonatmosphäre rühren und brach dann die Reaktion durch vorsichtige Zugabe von ca. 7 ml Eisessig ab. Das Reaktionsgemisch wurde in einen Scheidetrichter übergeführt, mit Dichlormethan auf das doppelte Volumen verdünnt und dreimal mit je 200 ml Wasser gewaschen, wobei die Wasserphasen jeweils noch einmal mit Dichlormethan extrahiert wurden. Die vereinigten organischen Phasen wurden über Watte filtriert und am Rotationsverdampfer vom Lösungsmittel befreit. Schließlich wurde der Rückstand im Feinvakuum bei 65° C von überschüssiger Essigsäure befreit. Man reinigte das Rohprodukt zunächst mittels Flash-Chromatographie (stationäre Phase: Matrex-Kieselgel, mobile Phase: Dichlormethan:Essigsäureethylester 6:1). Die Gesamtausbeute betrug 446 mg Isomerengemisch (89 % d. Th.).

Das so erhaltene Isomerengemisch wurde mittels präparativer Mitteldruck-Flüssigchromatographie (Säulenmaße: 460 mm Länge, 70 mm Innendurchmesser, mobile Phase: Dichlormethan:1,4-Dioxan 97:3, 20 ml/min) in insgesamt 15 Fraktionen aufgetrennt, die mittels analytischer HPLC (Polygosil 60-10, Dichlormethan:1,4-Dioxan 95:5, 2ml/min, UV 405 nm) zugeordnet wurden.

Man gab 1,03 g Isomerengemisch auf die Säule und isolierte 355 mg reines 3-Isomeres (Verbindung Nr. 4a) der Formel

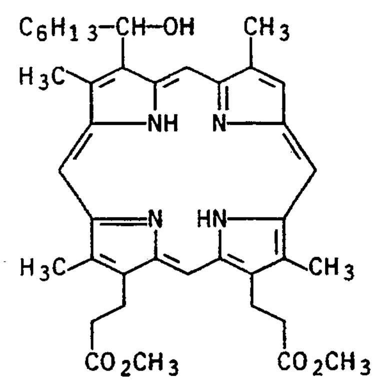

sowie 385 mg reines 8-Isomeres (Verbindung Nr. 4b) der Formel

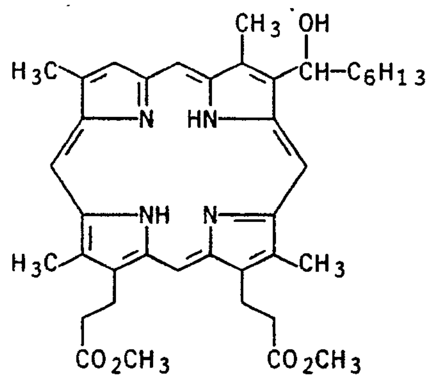

Für analytische Zwecke wurden reine Proben der Konstitutionsisomeren aus Dichlormethan/Methanol auskristallisiert.

Analytische Daten von Verbindung Nr. 4a

Fp: 155°C

UV/VIS (Chloroform): λmax(ε) = 621 nm (4700), 615 nm (sh, 3000), 574 nm (sh, 5800), 567 nm (6800), 531 nm (9400), 501 nm (sh, 14400), 498 nm (15000), 401 nm (182800), 377 nm (sh, 70000), 357 nm (sh, 35000), 327 nm (sh, 18600).

$^1$H-HMR (360 MHz, $CDCl_3$): δ = -3.95 (s, 2H, NH), 0.78 (t, $^3J$=6.5 Hz, 3H, Alk-$CH_3$), 1.18, 1.34 (2m, 8H, 3-$(CH_2)_4$), 1.64, 2.60 (2m, AB-Teil des ABX-Systems, 2H, 3-β-$CH_2$), 2.49 (m, 1H, R-OH), 3.25, 3.27 (2t, AA'-Teil des AA'MM'-Systems, $^3J$=7Hz, 4H, -OC(O)$CH_2$), 3.54, 3.57, 3.58, 3.64, 3.65, 3.69 (6s, 18H, 2,7,12,13,17,18-$CH_3$). 4.32, 4.34 (2t, MM'-Teil, $^3J$=7 Hz, 4H, 13,17-$CH_2$), 6.08 (t, X-Teil, $^3J$=7.2 Hz, 1H, 3-α-CH)-, 9.06 (s, 1H, 8-H), 9.97, 10.34 (2s, 4H, 5,10,15,20-H) ppm.

| | | | | |
|---|---|---|---|---|
| Analyse: $C_{39}H_{48}N_4O_5$ | ber.<br>gef. | C 71.75<br>C 71.71 | H 7.41<br>H 7.49 | N 8.58<br>N 8.63. |

Analytische Daten von Verbindung Nr. 4b

Fp: 173°C

UV/VIS (Chloroform): λmax(ε) = 621 nm (4400), 616 nm (sh, 2800), 573 nm (sh, 5500), 567 nm (7000), 532 nm (9300), 502 nm (sh, 14300), 498 nm (15100), 401 nm (185500), 378 nm (sh, 71000), 355 nm (sh, 34500), 326 nm (sh, 17000).

$^1$H-NMR (360 MHz, $CDCl_3$): δ = -3.97 (s, 2H, NH), 0.78 (t, $^3J$=7.2 Hz, 3H, Alkyl-$CH_3$), 1.19, 1.34 (2m, 8H, -$(CH_2)_4$-), 1.64, 2.59 (2m, AB-Teil des ABX-Systems, 2H, 8-β-$CH_2$), 2.48 (m, 1H, R-OH), 3.24, 3.25 (2t, AA'-Teil des AA'MM'-Systems, $^3J$=7,2Hz, 4H, 13,17-OC(O)$CH_2$), 3.51, 3.57, 3.58, 3.65, 3.66, 3.71 (6s, 18H, 2,7,12,13,17,18-$CH_3$). 4.34, 4.37 (2t, MM'-Teil, $^3J$=7.2 Hz, 4H, 13,17-$CH_2$), 6.05 (t, X-Teil, $^3J$=7.3 Hz, 1H, 8-α-CH)-, 9.04 (s, 1H, 3-H), 9.93, 9.99, 10.01, 10.28 (4s, 4H, 5,10,15,20-H) ppm.

| | | | | |
|---|---|---|---|---|
| Analyse: $C_{39}H_{48}N_4O_5$ | ber.<br>gef. | C 71.75<br>C 71.73 | H 7.41<br>H 7.51 | N 8.58<br>N 8.60. |

Beispiel 5

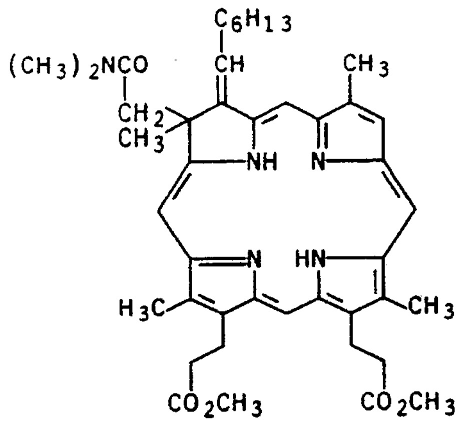

(Verbindung Nr. 5)

100 mg (0,154 mmol) der Verbindung Nr. 4a wurden in 10 ml 2-Xylol suspendiert und mit 2,6 ml (17,6 mmol) N,N-Dimethylacetamid-dimethylacetal versetzt. Man erhitzte in einer Argonatmosphäre unter Rückfluß am Wasserabscheider, der mit Molsieb (3Å) gefüllt war, wobei die Ölbadtemperatur im Verlauf einer Stunde von Raumtemperatur auf 160°C gesteigert und dann 90 Minuten bei dieser Temperatur belassen wurde.

Nach dem Abkühlen wurden die flüchtigen Bestandteile bei 70°C im Ölpumpenvakuum entfernt. Das schwarzgrüne Rohprodukt wurde mittels Flash-Chromatographie (stationäre Phase: Matrex-Kieselgel, mobile Phase: Dichlormethan:Essigsäureethylester 6:1) unter Lichtausschluß von Nebenprodukten befreit. Man erhielt 100,1 mg (90 % d. Th.) schwarzgrünes Produkt (Verbindung Nr. 5) in Form eines Gemisches der Konfigurationsisomeren (C-Atom 2:R- und S-Konfiguration; Doppelbindung an C-Atom 3:E- und Z-Konfiguration)

Fp.: 167°C.

UV/VIS (Chloroform): $\lambda max(\epsilon)$ = 659 nm (46600), 630 nm (3400), 603 nm (4600), 534 nm (13700), 504 nm (10800), 498 nm 11900), 401 nm (169300), 360 nm (sh, 32100), 344 nm (sh, 20000), 295 nm (13800).

$^1$H-NMR (360 MHz, $CDCl_3$): $\delta$ = -2.68, -2.52 (2s, 2H, NH), 0.96 (t, $^3J$=6.8 Hz, 3H, Alkenyl-$CH_3$), 1.48 (m, 4H, $(CH_2)_2$-), 1.80 (m, 2H, 3-$\delta$-$CH_2$), 1.96 (m, AA'-Teil des AA'MM'X-Systems, $^3J$=7Hz, 4H, 3-$\gamma$-$CH_2$), 2.20 (s, 3H, 2-$CH_3$), 2.27, 2.80 (2s, 6H, $NCH_3$), 2.95, 3.12 (2d, AB-System, $^2J$=14 Hz, 2H, 2-$CH_2CO$), 3.20 (m, AA'-Teil der AA'MM'-Systeme, 4H, 13,17-$CH_2$-$CH_2CO$), 3.30 (m, MM'-Teil des AA'MM'X-Systems, $^3J$=7 Hz, 2H, 3$\beta CH_2$), 3.41 (s, 3H, 12-$CH_3$), 3.47 (s, 3H, 18-$CH_3$), 3.58 (s, 3H, 7-$CH_3$), 3.66, 3.68 (2s, 6H, 13,17-$CH_3$), 4.18, 4.23 (2t, MM'-Teile der AA'MM'-Systeme, $^3J$=6.8 Hz, 4H, 13,17-$CH_2$), 6.28 (t, X-Teil, $^3J$=7 Hz, 1H, =C-H), 8.86 (s, 1H, 20-H), 8.94 (s, 1H, 8-H), 9.48 (s, 1H, 5-H), 9.69 (s, 1H, 15-H, 10-H); die Peaks entsprechen dem Z-Isomeren.

| Analyse: $C_{43}H_{55}N_5O_5$ | ber.<br>gef. | C 71.74<br>C 71.52 | H 7.68<br>H 7.76 | N 9.70<br>N 9.65. |
|---|---|---|---|---|

Beispiel 6

In analoger Weise wurden aus 110 mg (0,169 mmol) der Verbindung Nr. 4b 114 mg (94 % d. Th.) der Verbindung Nr. 6 der Formel

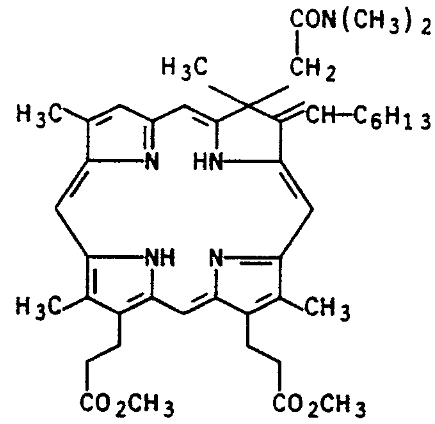

erhalten. Fp: 121 °C

UV/VIS (Chloroform): λmax(ε) = 659 nm (42200), 631 nm (3900), 603 nm (4600), 533 nm (12300), 503 nm (11300), 497 nm (12300), 486 nm (sh, 6000), 401 nm (181000), 360 nm (sh, 32100), 344 nm (sh, 21900), 296 nm (12500).

$^1$H-NMR (360 MHz, $CDCl_3$): Z-Isomer: $\delta$ = -2.56, (s, 2H, N-H), 0.97 (t, $^3J$=6.8 Hz, 3H, Alkenyl-$CH_3$), 1.47 (m, 4H, -$(CH_2)_2$-), 1.69 (m, 2H, 8-δ-$CH_2$), 1.97 (m, AA'-Teil des AA'MM'X-Systems, 2H, 8-γ-$CH_2$), 2.10 (s, 3H, 7-$CH_3$), 2.27, 2.79 (2s, 6H, $NCH_3$), 3.11-3.26 (m, 6H, 7,13,17-$CH_2CO$), 3.31 (m, MM'-Teil des AA'MM'X-Systems, $^3J$=7 Hz, 2H, 8-β-$CH_2$), 3.43 (s, 3H, 12-$CH_3$), 3.50 (s, 3H, 18-$CH_3$), 3.63 (s, 3H, 2-$CH_3$), 3.67, 3.69 (2s, 6H, 13,17-$CH_3$), 4.21, 4.37 (2t, 6H, $^3J$=7. Hz, 4H, 13,17-$CH_2$), 6.30 (t, X-Teil, $^3J$=6.8 Hz, 1H, =C-H), 8.68 (s, 1H, 3-H), 8.80 (s, 1H, 5-H), 9.47 (s, 1H, 10-H), 9.74 (2s, 2H, 15,20-H).

| | | | | |
|---|---|---|---|---|
| Analyse: $C_{43}H_{55}N_5O_5$ | ber. | C 71.54 | H 7.68 | N 9.70 |
| | gef. | C 71.59 | H 7.77 | N 9.60. |

Beispiel 7

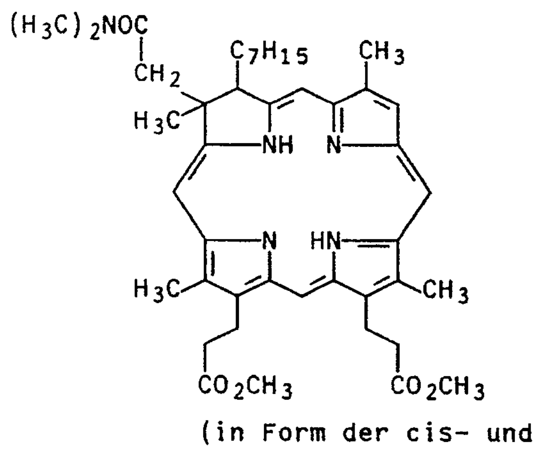

(in Form der cis- und trans-Verbindung)

55 mg (0,076 mmol) Verbindung Nr. 5 wurden in ca. 50 ml entgaster Ameisensäure gelöst und mit 25 mg $PtO_2$ (in 2 ml entgaster Ameisensäure suspendiert) versetzt. Man spülte die Apparatur dreimal mit Wasserstoff und ließ bei Normaldruck und Raumtemperatur zwei Stunden intensiv schütteln. Dann überführte man das tiefblaue Reaktionsgemisch in einen Scheidetrichter, extrahierte mit Dichlormethan, wusch mit gesättigter Natriumhydrogencarbonatlösung - wobei Farbumschlag nach Grün eintrat - und mit gesättigter Natriumchloridlösung. Die vereinigten organischen Phasen wurden durch Filtration über Watte getrocknet und am Rotationsverdampfer vom Lösungsmittel befreit. Den Rückstand reinigte man mittels Flash-Chromatographie (Säulenmaße: 15 cm Länge, 20 mm Innendurchmesser, stationäre Phase: Matrex-Kieselgel, mobile Phase: Dichlormethan-Essigsäureethylester 6:1). Man isolierte zwei Fraktionen, welche die beiden Diastereomeren enthielten (Isomerenverhältnis cis/trans: 1:1). Das cis-Isomer verließ die Säule zuerst. Man erhielt 22,6 mg (41 % d. Th.) cis-Isomer und 23.7 mg (43 % d. Th.) trans-Isomer. Zu analytischen Zwecken kristallisierte man kleine Proben aus Dichlormethan/n-Hexan um.

Analytische Daten für die cis-Verbindung ($C_7H_{15}$ und $(CH_3)_2NCO-CH_2$ sind cis-ständig):
Fp.: 155 °C
UV/VIS (Chloroform): $\lambda_{max}(\epsilon)$ = 645 nm (49100), 618 nm (3600), 593 nm (4100), 524 nm (3200), 497 nm (12500), 490 nm (12600), 393 nm (172800), 352 nm (sh, 32700), 334 nm (sh, 17500).
$^1$H-NMR (360 MHz, $CDCl_3$): $\delta$ = -2.52 (s, 2H, N-H), 0.87 (t, $^3J$=6.8 Hz, 3H, Alkyl-$CH_3$), 1.29, 1.47 (2m, 6H+2H, -$(CH_2)_4$-), 1.70 (m, AA'-Teil des AA'MNX-Systems, 2H, 3-$\beta$-$CH_2$), 1.35, 2.71 (2s, 6H, $NCH_3$), 2.26 (s, 3H, 2-$CH_3$), 2.41 (m, MN-Teil, 2H, 3-$\alpha$-$CH_2$), 3.13, 3.15 (2d, AB-System, $^2J$=14 Hz, 2H, 2-$CH_2CO$), 3.17, 3.18 (2t, AA'-Teile der AA'MM'-Systeme, $^3J$=8 Hz, 4H, 13,17-$CH_2CO$), 3.41 (s, 3H, 12-$CH_3$), 3.45 (s, 3H, 18-$CH_3$), 3.56 (s, 3H, 7-$CH_3$), 3.65, 3.69 (2s, 6H, 13,17-$CH_3$), 4.19 (t, MM'-Teil, $^3J$=8 Hz, 2H, 13-$CH_3$), 4.33, 4.34 (2t, MM'-Teil, $^3J$=8 Hz, 2H, 17-$CH_2$), 4.69 (t, X-Teil, $^3J$=6.8 Hz, 1H, 3-H), 8.79 (s, 1H, 20-H), 8.92 (s, 1H, 8-H), 8.99 (s, 1H, 5-H), 9.68 (s, 1H, 10-H), 9.72 (s, 1H, 15-H) ppm.

| | | | | |
|---|---|---|---|---|
| Analyse: $C_{43}H_{57}N_5O_5$ | ber. | C 71.34 | N 7.94 | H 9.67 |
| | gef. | C 71.54 | N 7.97 | H 9.59 |

Analytische Daten für die trans-Verbindung ($C_7H_{15}$ und $(CH_3)_2NCO-CH_2$ sind trans-ständig):
Fp.: 158 °C
UV/VIS (Chloroform): $\lambda_{max}(\epsilon)$ = 644 nm (42200), 617 nm (3900), 592 nm (4500), 524 nm (4100), 495 nm (12000), 491 nm (12000), 393 nm (151900), 351 nm (sh, 31900), 334 nm (sh, 20000).
$^1$H-NMR (360 MHz, $CDCl_3$): $\delta$ = -2.56 (s, 2H, N-H), 0.72 (t, $^3J$=6.6 Hz, 3H, Alkyl-$CH_3$), 1.0-1.4 (2m, u.a. AA'-Teil des AA'MNX-Systems, 10H, -$(CH_2)_5$-), 1.58, 2.64 (2s, 6H, $NCH_3$), 1.92, 2.28 (2m, MN-Teil, 2H, 3-$\alpha$-$CH_2$), 2.48 (s, 3H, 2-$CH_3$), 2.71, 3.02 (2d, AB-System, $^2J$=14.4 Hz, 2H, 2-$CH_2CO$), 3.16, 3.20 (2t, AA'-Teile der AA'MM'-Systeme, $^3J$=6.8 Hz, 4H, 13,17-$CH_2CO$), 3.41 (s, 3H, 12-$CH_3$), 3,48 (s, 3H, 18-$CH_3$), 3.54 (s, 3H, 7-$CH_3$), 3.67, 3.68 (2s, 6H, 13,17-$CH_3$), 4.20 (t, MM'-Teil, $^3J$=8.6 Hz, 2H, 13-$CH_2$), 4.33, 5.35 (2t, MM'-Teil, $^3J$=8.6 Hz, 2H, 17-$CH_2$), 4.88 (t, X-Teil, $^3J$=6.3 Hz, 1H, 3-H), 8.81 (s, 1H, 20-H), 8.92 (s, 1H, 8-H), 8.98 (s, 1H, 5-H), 9.68 (s, 1H, 10-H), 9.73 (s, 1H, 15-H) ppm.

Beispiel 8

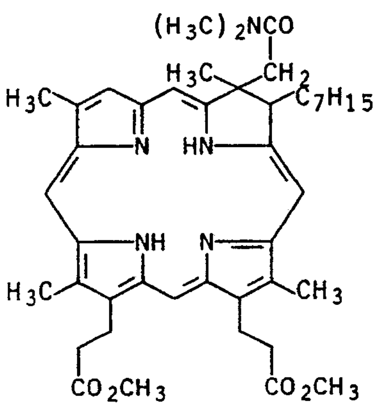

(in Form der cis- und trans-Verbindung)

Die Hydrierung wurde analog Beispiel 7 mit 55 mg (0,076 mmol) Verbindung Nr. 6 durchgeführt. Mittels Flash-Chromatographie isolierte man zwei Fraktionen, welche die beiden Diastereomeren enthielten (Isomerenverhältnis cis/trans: 1:2). Die Gesamtausbeute betrug 44,6 mg (81 % d. Th.), wobei 16,4 mg cis-Isomer und 28,2 mg trans-Isomer anfielen.

Analytische Daten für die cis-Verbindung ($C_7H_{15}$ und $(CH_3)_2NCO-CH_2$ sind cis-ständig):
Fp.: 223 °C
UV/VIS (Chloroform): $\lambda_{max}(\epsilon)$ = 643 nm (45000), 616 nm (4300), 591 nm (4800), 523 nm (3800), 496 nm

(13400), 490 nm (13400), 393 nm (183700), 353 nm (sh, 32600), 334 nm (sh, 19000).
$^1$H-NMR (360 MHz, $CDCl_3$): $\delta$ = -2.42 (s, 2H, N-H), 0.85 (t, $^3J$=6.8 Hz, 3H, Alkyl-$CH_3$), 1.28, 1.43 (2m, 6H+2H, $-(CH_2)_4-$), 1.65 (m, 2H, AA'-Teil des AA'MNX-Systems, 8-$\beta$-$CH_2$), 1.51, 2.74 (2s, 6H, $NCH_3$), 2.19 (s, 3H, 7-$CH_3$), 2.37 (m, MN-Teil des AA'MNX-Systems, 2 AA'-Teile der beiden AA'MM'-Systeme, $^3J$=7,7 Hz, 4H, 13,17-$CH_2CO$), 3.43, (s, 3H, 12-$CH_3$), 3.47 (s, 3H, 18-$CH_3$) 3.62 (s, 3H, 2-$CH_3$), 3.68, 3.69 (2s, 6H, 13,18-$CH_3$), 4.19, 4.32 (2t, MM'-Teile der AA'MM'-Systeme, $^3J$=7.7 Hz, 4H, 13,17-$CH_2$), 4,68 (t, X-Teil, $^3J$=6.2 Hz, 1H, 8-H), 8.62 (s, 1H, 3-H), 8.70 (s, 1H, 5-H), 8.94 (s, 1H, 10-H), 9.70, 9.75 (2s, 2H, 15,20-H) ppm.

| | | | | |
|---|---|---|---|---|
| Analyse: $C_{43}H_{57}N_5O_5$ | ber. | C 71.34 | N 7.94 | H 9.67 |
| | gef. | C 71.27 | N 7.95 | H 9.74 |

Analytische Daten für die trans-Verbindung ($C_7H_{15}$ und $(CH_3)_2NCO-CH_2$ sind trans-ständig):
Fp.: 132° C
UV/VIS (Chloroform): $\lambda_{max}(\epsilon)$ = 643 nm (43500), 616 nm (3200), 590 nm (3700), 523 nm (3100), 495 nm (11900), 490 nm (11900), 393 nm (178000), 352 nm (sh, 29800), 336 nm (sh, 18200).
$^1$H-NMR (360 MHz, $CDCl_3$): $\delta$ = -2.49, -2.40 (2s, 2H, N-H), 0.72 (t, $^3J$=6.8 Hz, 3H, Alkyl-$CH_3$), 1.00-1.36 (2m, u.a. AA'-Teil des AA'MNX-Systems, 10H, $-(CH_2)_5-$), 1.55, 2.62 (2s, 6H, $NCH_3$), 1.90, 2.25 (2m, MN-Teil des AA'MNX-Systems, 2H, 8-$\alpha$-$CH_2$), 2.44 (s, 3H, 7-$CH_3$), 2.70, 3.01 (2d, AB-System, $^2J$=14,8 Hz, 2H, 7-$CH_2CO$), 3.17, 3.21 (2t, AA'-Teile der beiden AA'MM'-Systeme, $^3J$=8,6 Hz, 4H, 13,17-$CH_2CO$), 3.43 (s, 3H, 12-$CH_3$), 3.45 (s, 3H, 18-$CH_3$), 3.62, (s, 3H, 2-$CH_3$), 3.68 (s, 6H, 13,17-$CH_3$), 4.24, 4.32 (2t, MM'-Teile der AA'MM'-Systeme, $^3J$=8.6 Hz, 4H, 13,17-$CH_2$), 4.86 (t, X-Teil, $^3J$=6.2 Hz, 1H, 8-H), 8.63 (s, 1H, 3-H), 8.70 (s, 1H, 5-H), 8.94 (s, 1H, 10-H), 9.70, 9.75 (2s, 2H, 15,20-H) ppm.

Beispiel 9

Verseifung des Dimethylesters aus Beispiel 7 (trans-Verbindung)

10 mg (0,014 mmol) der Verbindung aus Beispiel 7 (trans-Verbindung) wurden in 10 ml entgastem trockenem Tetrahydrofuran gelöst und mit 10 ml 5 M wäßriger Kalilauge versetzt. Das Reaktionsgemisch wurde in einer Argonatmosphäre bei 50° C 40 Stunden intensiv gerührt. Man überführte die Mischung in einen Scheidetrichter, stellte mit 1 M Salzsäure einen pH-Wert von ca. 3 ein und extrahierte erschöpfend mit Methyl-tert-butylether. Die vereinigten organischen Phasen wurden durch Filtration über Watte getrocknet und am Rotationsverdampfer vom Lösungsmittel befreit. Der grüne Rückstand wurde mittels Flash-Chromatographie (stationäre Phase: Matrex-Kieselgel, mobile Phase: Dichlormethan:Methanol 95:5) gereinigt. Man erhielt 7,9 mg (81 % d. Th.) der entsprechenden Dicarbonsäure.
Fp. 350° C.
UV/VIS (Chloroform): $\lambda_{max}(\epsilon)$ = 645 nm (24600), 619 nm (2900), 592 nm (2700), 523 nm (2300), 497 nm (7000), 492 nm (7000), 392 nm (90700).

Beispiel 10

Verseifung des Dimethylesters aus Beispiel 8 (trans-Verbindung)

10 mg (0,014 mmol) der Verbindung aus Beispiel 8 (trans-Verbindung) wurden analog Beispiel 9 umgesetzt und gereinigt. Man erhielt 7,4 mg (76 % d. Th.) der entsprechenden Dicarbonsäure.
Fp: 112-15° C.
UV/VIS (Chloroform): $\lambda_{max}(\epsilon)$ = 644 nm (19800), 616 nm (1600), 591 nm (1800), 524 nm (1500), 496 nm (5600), 491 nm (5600), 394 nm (83400).

**Patentansprüche**

1. Chlorinderivate der Formel I

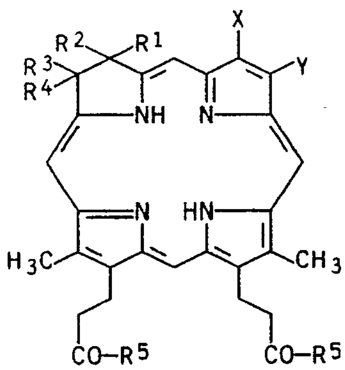

(I),

in der

eines der beiden Restepaare $R^1/R^2$ und $R^3/R^4$ Wasserstoff und $C_3$-$C_{12}$-Alkyl und das andere Methyl und $CH_2$-CO-$R^6$,

$R^5$ und $R^6$ gleich oder verschieden sind und unabhängig voneinander jeweils Hydroxy, $C_1$-$C_6$-Alkoxy, Amino oder $C_1$-$C_6$-Mono- oder Dialkylamino und

einer der beiden Reste X und Y Wasserstoff und der andere Methyl bedeuten.

**2.** Chlorinderivate nach Anspruch 1, die der Formel Ia

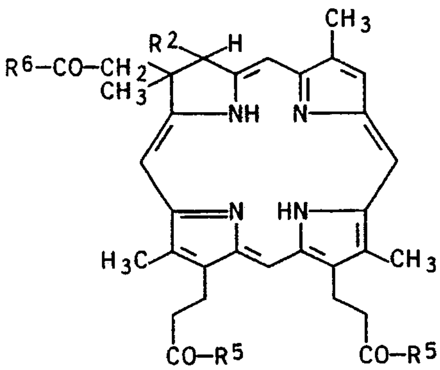

(Ia)

entsprechen, in der

$R^2$ $C_3$-$C_{12}$-Alkyl und

$R^5$ und $R^6$ gleich oder verschieden sind und unabhängig voneinander jeweils Hydroxy, $C_1$-$C_6$-Alkoxy, Amino oder $C_1$-$C_6$-Mono- oder Dialkylamino

bedeuten.

**3.** Chlorinderivate nach Anspruch 2, dadurch gekennzeichnet, daß

$R^2$ $C_5$-$C_{12}$-Alkyl und

$R^5$ und $R^6$ unabhängig voneinander jeweils Hydroxy, $C_1$-$C_4$-Alkoxy oder $C_1$-$C_4$-Dialkylamino

bedeuten.